# EUROPEAN PATENT SPECIFICATION

(11) **EP 1 246 573 B1**
(45) Date of publication and mention of the grant of the patent: **10.12.2008**
(21) Application number: 00989482.5
(22) Date of filing: 22.12.2000
(51) Int. Cl.: A61B 17/08, A61B 17/11

(54) **SOFT TISSUE STRUCTURE REPAIR USING ADHESIVE**
WEICHGEWEBEREPARATUR UNTER VERWENDUNG EINES KLEBERS
REPARATION DE STRUCTURE DE TISSU MOU A L'AIDE D'UN ADHESIF

(30) Priority: 23.12.1999 US 171963 P; 18.01.2000 US 176957 P
(43) Date of publication of application: 09.10.2002
(73) Proprietor: Omeros Corporation, Seattle, WA 98101 (US)
(72) Inventor: DEMOPULOS, Gregory, A., Mercer Island, WA 98040 (US); REED, Scott, W., Monroe, Connecticut 06468 (US); BACHMAN, Alan, B., New Haven, CT 06513 (US); KARL, Frederick, T., Newtown CT 06470-2643 (US); ALLEN, William, J., Stratford, CT 06614 (US); ADAMS, Leland, R., Ansonia, CT 06401 (US); THATCHER, Lawrence, G., Chelmsford, MA 01824 (US)
(74) Representative: Taylor, Anne Janette
(86) International application number: PCT/US2000/035261
(87) International publication number: WO 2001/045549

(56) References cited:
- US-A- 3 716 058
- US-A- 5 061 283
- US-A- 5 222 964
- US-A- 5 354 305
- US-A- 5 900 245
- US-A- 5 972 371

## Description

### Field of the Invention

The present invention relates to a system for repairing lacerated or severed soft tissue structures of the body, particularly connective cords such as tendons.

### Background of the Invention

Repair techniques for lacerated or severed tendons vary widely depending on the nature of the injury and the particular tendon affected. There are large differences in the extent to which access can be obtained in the least obtrusive manner, in the amount of tendon excursion, in the surrounding environment, in the stresses to which different tendons are normally subjected, and in the healing characteristics of different tendons. In addition, often there is no consensus of the overall best way to repair a given tendon.

For example, repair of a long flexor tendon in the hand that has been severed is typically achieved by suturing the severed tendon ends face-to-face. Historically, the joints across which the tendon acts were immobilized for from three to eight weeks to protect the tendon while it healed, because a freshly sutured tendon can withstand only a fraction of the tensile force to which a healthy tendon is subjected during normal use. Immobilization results in scarring and adhesion formation along the length of the tendon. Range of motion is adversely affected, particularly in the case of flexor tendons which normally glide smoothly through and over the unique system of tendon tunnels and pulleys of the hand. Nevertheless, it was thought that fibroblastic ingrowth was required in order for the tendon to heal, such that immobilization and the resulting decreased range of motion were considered necessary evils in order for effective healing to take place. More recently it has been discovered that flexor tendons have an intrinsic capacity to heal and at early motion may actually expedite healing. Still, exercises must be carefully planned and carried out due to the weakness of the sutured repair. In early stages of healing, protected passive and/or restricted active exercises may be used, followed by tendon gliding and active strengthening exercises in later stages. The affected joints are most often partially immobilized to prevent inadvertent application of excess force.

US Patent No 5 061 283 discloses devices for repairing severed tendons or ligaments by suturing opposing ends of the severed tendon or ligament. US Patent No 5 972 371 describes a tube possessing holes to permit suturing of severed ends of nerves, tendons or muscles inserted into the tube.

### Summary of the Invention

The present invention is concerned with a system for repair of injured soft tissue structures, particularly connective cords such as tendons, by use of adhesive and, preferably, reinforcing members or stents which can be made of substantially rigid or semi-rigid material. Such stents are adapted for extending longitudinally between severed end portions of a tendon with the severed end portions in abutting relationship. The tendon is secured to the stent by the adhesive such that tension applied to the tendon is transmitted through the stent. The stent and adhesive maintain the severed tendon ends abutting as tension is applied to the tendon.

### Brief Description of the Drawings

The foregoing aspects and many of the attendant advantages of this invention will become more readily appreciated as the same become better understood by reference to the following detailed description, when taken in conjunction with the accompanying drawings, wherein:
FIGURE 1 is a diagrammatic top perspective of a severed end of a tendon and a cutting tool that can be used to prepare a tendon for repair using the present invention;
FIGURE 2 is a top perspective corresponding to FIGURE 1 showing a severed tendon is the process of repair in accordance with the present invention;
FIGURE 3 is a top perspective of another tool that can be used when repairing a tendon in accordance with the present invention;
FIGURE 4 is a top perspective of a first embodiment of a stent usable in the present invention;
FIGURE 5 is an enlarged top perspective of a second embodiment of a stent usable in the present invention;
FIGURE 6 is a diagrammatic top perspective of a severed tendon in the proces of repair in accordance with the present invention; and
FIGURE 7 is side elevation of the repair of FIGURE 6, with parts broken away,

### Detailed Description of the Preferred Embodiment

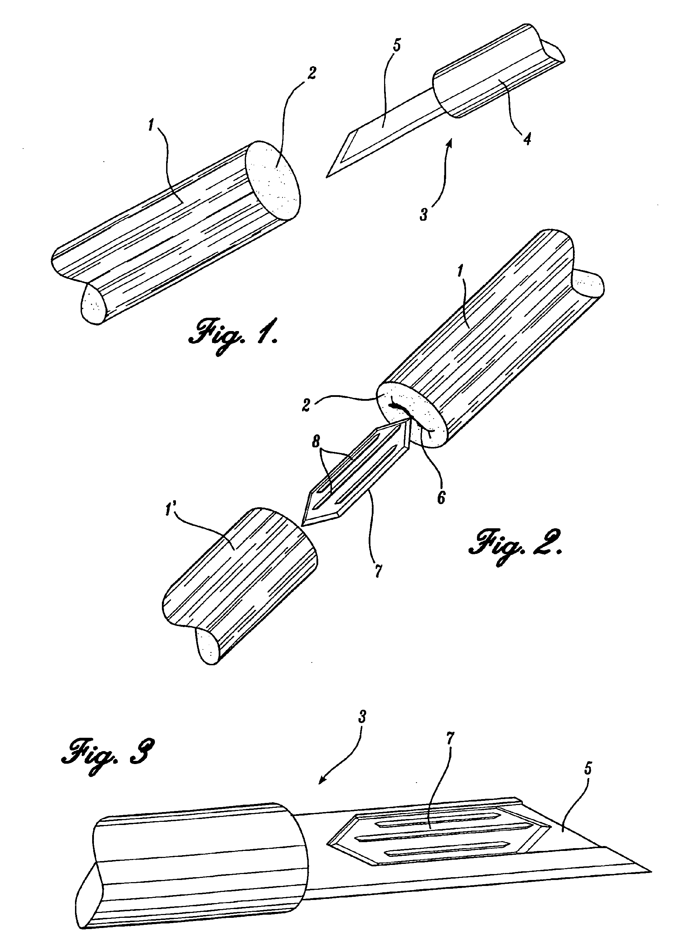

The present invention provides a splice for soft tissue structure having a laceration. FIGURE 1 shows a tendon 1 severed at a lacerated "end" face 2. In preparation for insertion of a reinforcing member or stent, a cutting tool 3 having a handle 4 and distally projecting blade 5 can be used to cut a slit 6 (FIGURE 2) in the end face 2 of the tendon 1. Preferably the slit does not extend the full width of the tendon, so that the external sheath of the tendon is not weakened. The same procedure can be used for forming a slit in the mating tendon end portion 1' (see FIGURE 2).

Next, a reinforcing member or stent 7 is inserted into first one then the other slit 6, and the end faces of the severed tendon 1, 1' are brought together to an abutting relationship. The stent 7 shown in FIGURE 2 is much longer than it is wide and much wider than it is thick, having opposite broad surfaces at the top and bottom. More specifically, the width of the stent is approximately equal to the width of the preformed slits 6, and the length of the stent is approximately equal to the depth of the slits measured axially or lengthwise of each tendon portion 1, 1 The stent may be inserted by use of a suitable grasping tool, eased by forming the stent with pointed and/or sharpened ends. Alternatively, as represented diagrammatically in FIGURE 3, the blade 5 of the cutting tool 3 can be formed with a depression for receiving the stent 7. By careful manipulation of the cutting tool, the stent may be automatically deposited in a first end portion 1, 1' of the tendon as the cutting tool is removed following formation of the slit 6. Another alternative is to form the slit with one tool, and deposit the stent with another tool similar to that shown in FIGURE 3.

Ultimately, the stent will be held in place within the slits 6 by adhesive. To increase the surface area between the stent, the adhesive and the tendon, and to form channels for receiving the adhesive, the stent 7 of FIGURE 2 may have longitudinally extending grooves 8 to expand surface area in both its upper and lower surfaces. These grooves extend only partway into the stent to isolate the top surface from the bottom.

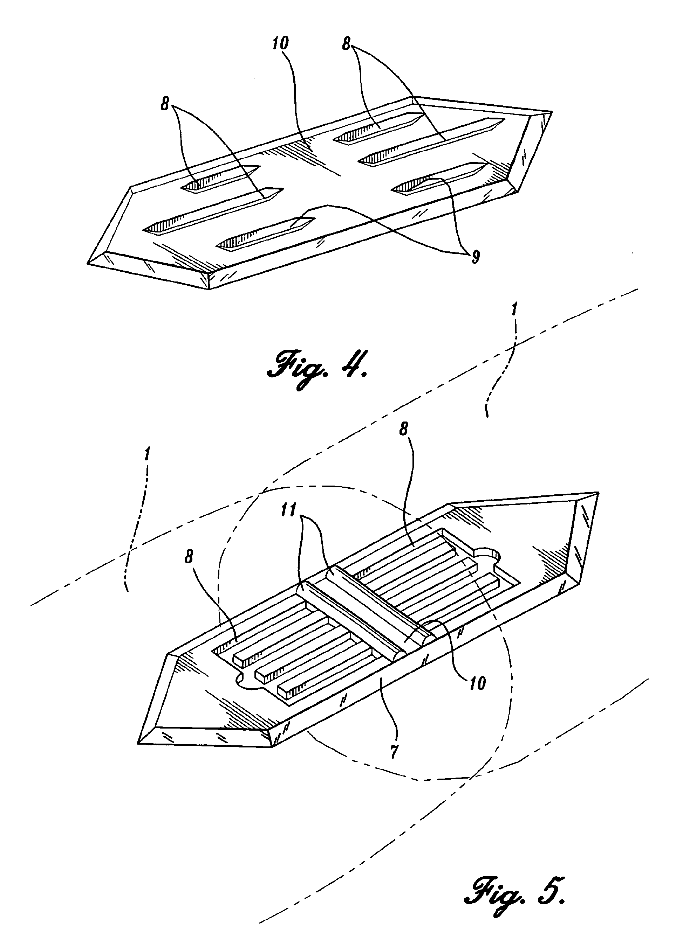

With reference to FIGURE 4, it may be desirable to prevent the adhesive from flowing between the abutting faces of the severed tendon, Consequently, the grooves 8 can have inner ends 9 which are spaced apart, such that an ungrooved area 10 is formed at the center of the stent, both in the top and bottom surfaces, in the area where the stent bridges between the abutting faces of the severed tendon.

FIGURE 5 shows another construction in more detail, including a pattern of grooves 8 at the opposite end portions of the stent 7. At each end the grooves are in communication with each other, but the grooves at one end are not in communication with the grooves at the other. Short bumps 11 can be formed at the inner ends of the grooves 8 at each end, to further isolate the central portion 10 from the grooves and, preferably, prevent adhesive from flowing to the abutting faces of the severed tendon. While the grooves 8 can be formed in only one or the other of the flat surfaces of the stent 7, preferably the grooves are formed both at the top and at the bottom.

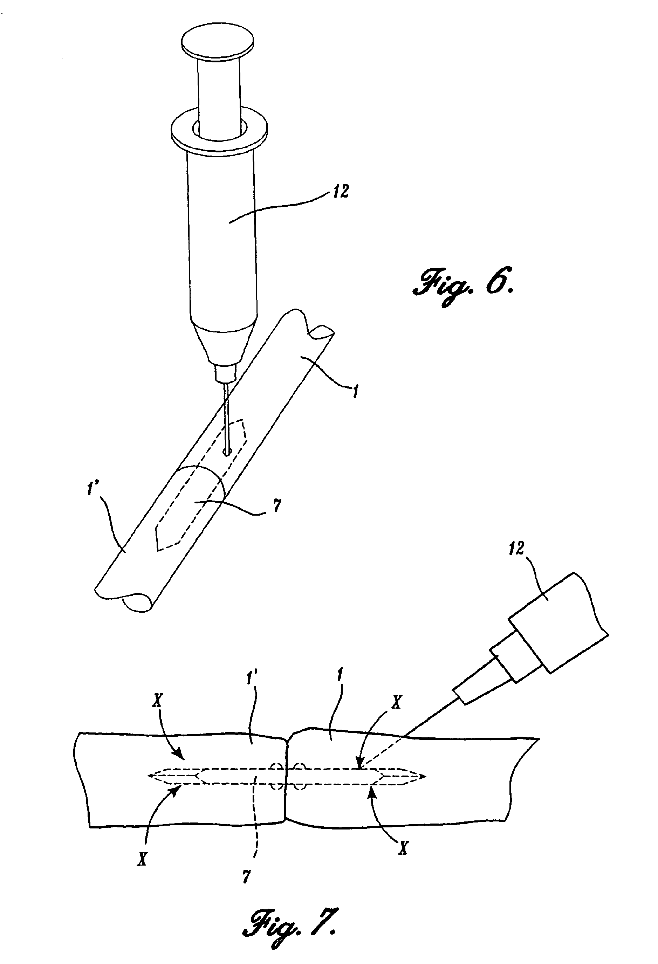

With reference to FIGURE 6 and FIGURE 7, a settable liquid adhesive is used to fix the stent within the slits of the severed tendon end portions 1, 1'. One way this can be done is by use of a syringe 12. For consistent results, the amount of adhesive is predetermined, i.e., a "metered" amount of injected adhesive is used. As illustrated diagrammatically in FIGURE 7, the adhesive can be injected by use of the syringe at a plurality of sites along the stent (e.g., four locations designated "x", two at each end, one of which is at the top surface and the other of which is at the bottom surface). In addition, to provide additional strength of the repair and a smooth outer profile of the repaired tendon, a metered quantity of an adhesive can be carefully applied externally around the area of the abutting faces of the severed tendon, preferably without injecting or disbursing that adhesive between the severed ends which could interfere with healing.

Preferably the stent will be dimensionally stable so that once it is secured in place it will not elongate or stretch which could alter the abutting relationship of the severed tendon ends and interfere with healing. Nevertheless, the stent should be reasonably flexible, possibly approaching the flexibility of the tendon itself, so as not to interfere with excursion of the tendon during normal motion. This may require that the stent be very thin. Widthwise and lengthwise, the stent has substantial dimensions for increasing the effective surface area for bonding the stent internally of the tendon by use of the adhesive.

It is envisioned that the adhesive can be of the general type described in U.S. Patent No. 5,350,798 of Linden et al. or a variant. Such an adhesive is, in general, a polymer gel and, more specifically, a cyanoacrylate polymer. Modified gels are described in U.S. Patent Nos. 5,714,159 and 5,612,052 of Shalaby.

At the time of injection, preferably the adhesive flows freely without high adhesive properties relative to the tendon, but will thereafter set quickly and secure the severed tendon ends in the desired abutting relationship. The adhesive preferably will have a high sheer strength and approximately the same bending and deflection characteristics as the stent, i.e., the adhesive, once set, will not stretch substantially and also will not be so rigid as to crack if the repaired tendon undergoes normal deflection. The adhesive may inherently have disinfectant characteristics and/or may be coated or impregnated with a compound having disinfectant characteristics. Alternatively or additionally, the adhesive may serve as a delivery system for drugs and/or agents and/or factors to promote healing and/or growth. Both the stent and the adhesive preferably are bioabsorbable, but over a sufficiently long length of time that full healing of the tendon occurs. Materials currently under consideration for the stent are E-caprolactone and poly-L-lactide, or a blend or co-polymer of E-caprolactone and/or trimethylene carbonate and poly-L-lactide, preferably with an inherent viscosity in the range of 1.0 to 2.8 dL/gm at 20°C. These polymers also may be optimized to meet the physical requirements of a successful tendon repair, such as by controlling the degree of crystallinity via primary and/or secondary processing conditions. In general, it is preferred that the strength of the stent-adhesive repair plus the strength of the healed or partially healed tendon be equal or greater to that necessary for full active motion of the tendon. For example, in the case of a flexor tendon of the hand, the stent-adhesive repair may provide "full" strength for approximately three weeks and at least about 50% strength at six weeks when the partially healed tendon itself has 50% or more of its normal tensile strength.

For a typical repair of a flexor tendon of the hand, the width of the stent could be about 3 mm, the length about 2 to 3 cm and the thickness about 0,7 mm in order to withstand a force of 5000 to 6500 grams without substantial stretching or elongation. This results in a bonding area at each side of at least about 3 mm by 10 mm at both the top surface and the bottom surface, although the actual size of the bonding area will depend on the design of the stent. The bond strength at each of the four bonding areas (top and bottom at each side of the tendon laceration) is preferably at least about 2500 to about 3250 grams.

## Claims

1. A splice for soft tissue structure having a laceration forming adjacent lacerated surfaces (2), said splice being an elongated reinforcing member (7), the reinforcing member being substantially longer than it is wide and substantially wider than it is thick defining opposite broad surfaces, the reinforcing member (7) being sized for insertion into the soft tissue structure in an orientation extending across the laceration with the lacerated surfaces abutting (2) and **characterized in that** the splice possesses grooves (8) to expand the surface area for being secured inside the soft tissue structure by a quantity of adhesive such that tension applied to the soft tissue structure is transmitted through the reinforcing member.

2. A splice according to claim 1 for a tendon (1) normally tensioned in the body during joint movement and having a laceration forming adjacent lacerated ends, the reinforcing member (7) being sized for insertion into the tendon in an orientation extending across the laceration with the lacerated ends (2) abutting and being adapted for being secured inside the tendon without penetrating the circumferential sheath of the tendon by a quantity of adhesive such that tension applied to the tendon (1) is transmitted through the reinforcing member.

3. A splice according to claim 1 or 2 in which the reinforcing member (7) is dimensionally stable in the direction of its length so as not to stretch during normal tensioning of the tendon (1), wherein optionally
the reinforcing member is flexible in a direction transverse to its length about an axis extending widthwise of the reinforcing member (7).

4. A splice according to any of claims 1-3 in which opposite end portions of the opposite broad surfaces of the reinforcing member have said grooves (8) for increasing the respective surface areas of such end portions, the reinforcing member having an ungrooved central portion positioned to be located in the area of abutting lacerated ends of the tendon and or/a splice
in which at least one of the opposite broad surfaces includes a central portion (10) positioned to be aligned with the abutting lacerated ends of the tendon and enlargements (11) formed at opposite sides of the central portion (10) to prevent flow of adhesive into the central portion.

5. A splice according to any of the preceding claims, in which the reinforcing member has sharpened opposite ends and/or
in which the reinforcing member has pointed opposite ends.

6. A splice according to any of the preceding claims for repair of a soft tissue structure having abutting lacerated surfaces (2), said splice comprising an elongated internal reinforcing member (7) bridging across the abutting lacerated surfaces for extending into the portions of the soft tissue structure adjacent to the lacerated surfaces (2), and being adapted to receive a quantity of adhesive for securing opposite end portions of the reinforcing member in the portions of the soft tissue structure adjacent to the lacerated surfaces (2) such that tension applied to the soft tissue structure is transmitted across the abutting surfaces (2) by way of the reinforcing member (7), the reinforcing member (7) being dimensionally stable in the direction of its length so as not to stretch upon application of normal tension to the soft tissue structure.

7. A splice according to any of the preceding claims for repair of a tendon having abutting lacerated ends (2), said splice comprising an elongated internal reinforcing member (7) bridging across the abutting lacerated ends for extending into the adjacent tendon end portions without penetrating the circumferential sheath of the tendon, and being adapted to receive a quantity of adhesive for securing opposite end portions of the reinforcing member (7) in the opposite lacerated end portions at opposite sides of the abutting ends (2) such that tension applied to the tendon is transmitted across the abutting ends (2) by way of the reinforcing member (7), the reinforcing member being dimensionally stable in the direction of its length so as not to stretch upon application of normal tension to the tendon.

8. A splice according to any of claim 6 or 7, in which the adhesive and reinforcing member are bioabsorbable over a period of time at least as great as the normal period for healing of the abutting lacerated ends of soft tissue or tendon.

9. A splice according to claim 8, in which the adhesive is a cyanoacrylate polymer and/or
the adhesive has disinfectant characteristics and/or
the adhesive contains an agent to promote healing.

## Patentansprüche

1. Eine Spleißverbindung für eine weiche Gewebestruktur, welche einen Riss aufweist, der gerissene Oberflächen (2) enthält, wobei die Spleißverbindung ein längliches Verstärkungselement (7) bildet, das wesentlich länger als breit ist und wesentlich breiter als dick ist und gegenüberliegende breite Oberflächen enthält, wobei das Verstärkungsglied (7) zum Einsatz in die weiche Gewebestruktur in einer Orientierung angepasst ist, die sich quer über den Riss mit den gerissenen aneinanderstoßenden Oberflächen (2) erstreckt, **dadurch gekennzeichnet, dass** die Spleißverbindung Vertiefungen (8) enthält, um den Oberflächenbereich der in der weichen Gewebestruktur durch eine Menge von Klebematerial gesichert ist, derart zu vergrößern, dass auf die weiche Gewebestruktur aufgebrachte Spannung über das Verstärkungsglied übertragen wird.

2. Spleißverbindung nach Anspruch 1 für eine Sehne (1), die normalerweise im Körper während einer gemeinsamen Bewegung gespannt wird und einen Riss aufweist, der gegenüberstehende gerissene Enden enthält, wobei das Verstärkungselement (7) so geformt ist, dass es in die Sehne in einer Orientierung quer zum Riss einsetzbar ist, wobei die gerissenen Enden (2) aneinanderstoßen und es in der Sehne gesichert wird, ohne dass die Umhüllung der Sehne durch eine Klebemenge durchdrungen wird, so dass auf die Sehne (1) aufgebrachte Spannung über das Verstärkungsglied übertragen wird.

3. Spleißverbindung nach Anspruch 1 oder 2, bei der das Verstäricungsglied (7) in Richtung der Länge dimensionsstabil ist, so dass es sich während einer normalen Spannung der Sehne (1) nicht dehnt, wobei optional das Verstärkungselement in Richtung quer zur Länge um eine Achse flexibel ist, die sich in der Breite des Verstärkungselements (7) erstreckt.

4. Spleißverbindung nach Anspruch 1 - 3, bei der gegenüberliegende Endbereiche der gegenüberliegenden breiten Oberflächen des Verstärkungselements die Vertiefungen (8) enthalten, um die entsprechenden Oberflächenbereiche solcher Endbereiche zu vergrößern, wobei das Verstärkungselement einen nicht mit einer Vertiefung versehenen zentralen Bereich aufweist, der in dem Bereich der aneinanderstoßenden gerissenen Enden der Sehne ausgebildet ist und/oder eine Spleißverbindung, bei der wenigstens eine der gegenüberliegenden breiten Flächen einen zentralen Bereich (10) aufweist, der so angeordnet ist, dass er mit den gegenüberliegenden gerissenen Enden der Sehne ausgerichtet ist und bei der Vergrößerungen (11) an gegenüberliegenden Seiten des zentralen Bereichs (10) vorgesehen sind, um den Klebemittelfluss in den zentralen Bereich zu verhindern.

5. Spleißverbindung nach einem der vorhergehenden Ansprüche, bei der das Verstärkungselement scharfe gegenüberliegende Enden aufweist und/oder bei der das Verstärkungselement gespitzte gegenüberliegende Enden aufweist.

6. Spleißverbindung nach einem der vorhergehenden Ansprüche zur Reparatur einer weichen Gewebestruktur, welche aneinanderstoßende gerissene Oberflächen (2) aufweist, wobei die Spleißverbindung aus einem länglichen internen Verstärkungselement (7) gebildet ist, das die aneinanderstoßenden gerissenen Oberflächen überbrückt, um in die Bereiche der weichen Gewebestruktur neben den gerissenen Oberflächen (2) einzudringen, und das so ausgebildet ist, dass es eine Klebstoffmenge zur Sicherung der gegenüberliegenden Endbereiche des Verstärkungselements in den an die gerissenen Oberflächen (2) angrenzenden Bereichen der weichen Gewebestruktur aufnehmen kann, derart, dass eine auf die weiche Gewebestruktur aufgebrachte Spannung über die aneinanderstoßenden Oberflächen (2) durch das Verstärkungselement (7) übertragen werden, wobei das Verstärkungselement (7) so dimensioniert ist, dass es in Richtung der Länge dimensionsstabil ist, so dass es sich bei Anwendung einer normalen Spannung auf die weiche Gewebestruktur nicht dehnt.

7. Spleißverbindung nach einem der vorhergehenden Ansprüche zur Reparatur einer Sehne mit gegenüberliegenden gerissenen Enden (2), wobei die Spleißverbindung aus einem länglichen internen Verstärkungselement (7) gebildet ist, das die aneinanderstoßenden gerissenen Enden überbrückt, um in das benachbarte Sehnenendteil einzudringen, ohne in die Ummantelung der Sehne einzudringen und das so ausgebildet ist, dass es eine Klebstoffmenge aufnimmt, um die gegenüberliegenden Endbereiche des Verstärkungselement (7) in den gegenüberstehenden gerissenen Endbereichen der gegenüberliegenden Seiten der aneinanderstoßenden Enden (2) derart zu sichern, dass auf die Sehne aufgebrachte Spannung über die gegeneinanderstoßenden Enden (2) über das Verstärkungselement (7) übertragen werden, wobei das Verstärkungselement (7) in Richtung seiner Länge dimensionsstabil ist, so dass es sich bei Aufbringung einer normalen Spannung auf die Sehne nicht dehnt.

8. Spleißverbindung nach einem der Ansprüche 6 oder 7, bei der der Klebstoff und das Verstärkungselement über einen Zeitraum bioabsorbierbar sind, der wenigstens so groß ist, wie der normale Zeitraum des Heilens aneinanderstoßender gerissener Enden einer weichen Gewebestruktur oder einer Sehne.

9. Spleißverbindung nach Anspruch 8, bei der der Klebstoff ein Cyanoacrylatpolymer ist und/oder der Klebstoff Desinfektionseigenschaften und/oder der Klebstoff ein Mittel zur Unterstützung des Heilens aufweist.

## Revendications

1. Dispositif de raccordement pour la structure d'un tissu mou ayant une lacération formant des surfaces adjacentes lacérées (2), ledit dispositif de raccordement étant un organe de renforcement allongé (7), l'organe de renforcement étant nettement plus long qu'il n'est large, et nettement plus large qu'il n'est épais, en délimitant des surfaces opposées larges, l'organe de renforcement (7) étant dimensionné pour une insertion dans la structure d'un tissu mou selon une orientation s'étendant à travers la lacération, les surfaces lacérées (2) étant en butée, et **caractérisé en ce que** le dispositif de raccordement comporte des gorges (8) afin d'étendre la surface pour le fixer à l'intérieur de la structure du tissu mou à l'aide d'une quantité d'adhésif, de telle façon qu'une tension appliquée à la structure du tissu mou soit transmise à travers l'organe de renforcement.

2. Dispositif de raccordement selon la revendication 1, pour un tendon (1) normalement tendu dans le corps au cours d'un mouvement d'articulation, et ayant une lacération formant des extrémités adjacentes lacérées, l'organe de renforcement (7) étant dimensionné pour une insertion dans le tendon selon une orientation s'étendant à travers la lacération, les extrémités lacérées (2) étant en butée, et étant adapté à être fixé à l'intérieur du tendon sans pénétrer dans la gaine circonférentielle du tendon, à l'aide d'une quantité d'adhésif, de telle façon qu'une tension appliquée au tendon (1) soit transmise à travers l'organe de renforcement.

3. Dispositif de raccordement selon la revendication 1 ou 2, dans lequel l'organe de renforcement (7) a une dimension stable dans la direction de sa longueur de façon à ne pas s'étirer au cours de la tension normale du tendon (1), dans lequel :
l'organe de renforcement est facultativement souple dans une direction transversale à sa longueur autour d'un axe s'étendant dans la direction de la largeur de l'organe de renforcement (7).

4. Dispositif de raccordement selon l'une quelconque des revendications 1 à 3, dans lequel les parties d'extrémité opposées des surfaces larges opposées de l'organe de renforcement comportent lesdites gorges (8) afin d'accroître les surfaces respectives de telles parties d'extrémité, l'organe de renforcement comportant une partie centrale sans gorge, positionnée de façon à être située dans la zone des extrémités lacérées en butée du tendon et/ou d'un raccord,
dans lequel au moins l'une des surfaces larges opposées comprend une partie centrale (10) positionnée de façon à être alignée avec les extrémités lacérées en butée du tendon, et des protubérances (11) formées à des côtés opposés de la partie centrale (10) pour prévenir l'écoulement de l'adhésif dans la partie centrale.

5. Dispositif de raccordement de l'une quelconque des revendications précédentes, dans lequel l'organe de renforcement a des extrémités opposées aiguisées et/ou dans lequel :
l'organe de renforcement a des extrémités opposées pointues.

6. Dispositif de raccordement selon l'une quelconque des revendications précédentes, pour la réparation d'une structure de tissu mou ayant des surfaces lacérées en butée (2), ledit dispositif de raccordement comprenant un organe de renforcement interne allongé (7) traversant les surfaces lacérées en butée pour s'étendre dans les parties de la structure de tissu mou adjacentes aux surfaces lacérées (2), et étant adapté à recevoir une quantité d'adhésif pour fixer des parties d'extrémité opposées de l'organe de renforcement dans les parties de la structure de tissu mou adjacentes aux surfaces lacérées (2) de telle façon qu'une tension appliquée à la structure de tissu mou soit transmise à travers les surfaces en butée (2) par l'intermédiaire de l'organe de renforcement (7), l'organe de renforcement (7) ayant une dimension stable dans la direction de sa longueur, de façon à ne pas s'étirer par application d'une tension normale à la structure de tissu mou.

7. Dispositif de raccordement selon l'une quelconque des revendications précédentes, pour la réparation d'un tendon comportant des extrémités lacérées en butée (2), ledit dispositif de raccordement comprenant un organe de renforcement interne allongé (7) traversant les extrémités lacérées en butée pour s'étendre dans les parties d'extrémité adjacentes du tendon sans pénétrer dans la gaine circonférentielle du tendon, et étant adapté à recevoir une quantité d'adhésif pour fixer des parties d'extrémité opposées de l'organe de renforcement (7) dans les parties d'extrémité lacérées opposées, aux faces opposées des extrémités en butée (2), par l'intermédiaire de l'organe de renforcement (7), l'organe de renforcement ayant une dimension stable dans la direction de sa longueur, de façon à ne pas s'étirer par application d'une tension normale au tendon.

8. Dispositif de raccordement selon l'une quelconque des revendications 6 et 7, dans lequel l'adhésif et l'organe de renforcement sont biorésorbables au cours d'une période au moins aussi longue que la période de cicatrisation des extrémités lacérées en butée du tissu mou ou du tendon.

9. Dispositif de raccordement selon la revendication 8, dans lequel l'adhésif est un polymère de cyanoacrylate et/ou
l'adhésif à des propriétés désinfectantes et/ou
l'adhésif contient un agent pour favoriser la cicatrisation.
